# EUROPEAN PATENT SPECIFICATION

(11) **EP 2 620 124 B1**
(45) Date of publication and mention of the grant of the patent: **18.05.2016**
(21) Application number: 13152795.4
(22) Date of filing: 25.01.2013
(51) Int. Cl.: A61D 1/02, A61M 5/315

(54) **Injection syringe**
Injektionsspritze
Seringue d'injection

(30) Priority: 27.01.2012 BE 201200055
(43) Date of publication of application: 31.07.2013
(73) Proprietor: Hubert De Backer NV, 9140 Temse (BE)
(72) Inventor: De Backer Jr., Jan, 9100 Sint-Niklaas (BE)
(74) Representative: Brantsandpatents bvba

(56) References cited:
- GB-A- 2 229 374
- US-A- 5 478 321

## Description

The invention concerns an injection syringe, in particular an intramammary injection syringe, comprising a first and a second reservoir, which first reservoir has a first open end through which the second reservoir can be mounted sliding in the first reservoir, which first reservoir has a second end located opposite the first end and on which an injection needle can be arranged, which second reservoir comprises a base surface and is provided with an outlet.

Such an intramammary injection syringe is known from US - PS 7 828 765. The known injection syringe is namely used in veterinary medicine, in particular in cows, to combat udder infections. The injection syringe comprises a first reservoir for the storage of a medication, in particular an antibiotic, and a second reservoir for storage of a salve. Because the second reservoir is mounted sliding in the first reservoir, by sliding the second reservoir into the first reservoir on administration to an animal, the content of the first reservoir first flows out via the injection needle. This is also important because the administration of the contents of the first and second reservoirs must take place sequentially and no mixing of substances may occur before they have been administered to the animal. For this only when the base surface of the second reservoir reaches the second end of the first reservoir is the outlet of the second reservoir then opened to administer its contents to the animal. In the known injection syringe, the outlet from the second reservoir is opened by means of a protrusion located in the base of the first reservoir in the extension of the injection needle. This protrusion presses away a plug which sits on the outlet of the second reservoir when the base surface of the second reservoir reaches this protrusion as a result of displacement into the first reservoir.

A disadvantage of the known intramammary injection syringe is on the one hand the presence of the protrusion in the base of the first reservoir and on the other hand the plug on the outlet of the second reservoir. Due to the presence of the protrusion, some medication always remains in the base of the first reservoir. This medication can then mix with the substance from the second reservoir which thereby can lose its effect on the animal. Mounting of the plug furthermore entails an extra production stage as this cannot take place in the same operation as casting of the second reservoir. The cost price for production of the known injection syringe is thus increased.

GB2229374 A discloses a two-compartment mixing dispenser having a cylinder with a chamber having a dispensing outlet and a secondary internal cylinder. US5478321 A discloses a prefilled disposable plastic syringe having a barrel end with a frangible closure integral with the needle fitting.

The object of the invention is to produce an injection syringe, in particular an intramammary injection syringe, which is cheap to produce and where in addition the evacuation of the first reservoir takes place as optimally as possible.

An injection syringe according to the invention is therefore characterised in that a protrusion is mounted at the outlet and in the mounted state of the injection syringe extends from the base surface towards the second end, which protrusion is mounted breakably on the base surface of second reservoir, such that when the protrusion reaches the second end, it breaks off to open the outlet of the second reservoir. Because the protrusion is arranged on the base surface of the second reservoir and breaks away on reaching the second end, it does not form an obstacle for the outflow of the medication from the first reservoir. The evacuation of the first reservoir is therefore optimum. Because in addition the protrusion is arranged at the outlet of the second reservoir in the base surface, this can take place at the same time as the production of the injection syringe without extra steps on injection moulding of the second reservoir.

The injection syringe according to the invention is characterised in that the protrusion is arranged offset in relation to a central axis of the injection syringe. Thus the force on breakage of the protrusion increases, whereby a more efficient breakage can occur.

Preferably the protrusion has a Y-shaped profile in cross-section. The advantage of such a profile is that it gives a certain strength and flexibility due to its geometry. Thus when the protrusion reaches the second end of the first reservoir, a suitable transition is formed between the second end and the base surface of the second reservoir, whereby a reliable breakage of the protrusion is achieved.

A second preferred embodiment of an injection syringe according to the invention is characterised in that that a circular weakness is made in the base surface which delimits a disc on which the Y-shaped profile is mounted. This makes breakage of the protrusion easier.

The third preferred embodiment of an injection syringe according to the invention is characterised in that the first and second reservoir are substantially cylindrical and that the second reservoir is fitted with a sealing element which is formed by a flexible ring arranged along a circumference of the casing surface of the second reservoir. Cylindrical reservoirs are comfortable to handle, easy to produce and in addition allow the sealing element to be produced as a ring.

A fourth preferred embodiment of an injection syringe according to the invention is characterised in that the base surface is surrounded by a skirt. This achieves the protection of the base surface.

The invention will now be described in more detail with reference to the drawing which shows an embodiment example of an injection syringe according to the invention. The drawing shows:
Figure 1 an overall view of an injection syringe according to the invention;
Figure 2 a cross-section in longitudinal direction through the first reservoir of the injection syringe;
Figure 3 a cross-section in longitudinal direction through the injection syringe when the reservoirs are full;
Figure 4 a cross-section in longitudinal direction through the injection syringe when the first reservoir is almost empty;
Figure 5 a cross-section in longitudinal direction through the injection syringe when the first reservoir is totally empty; and
Figure 6 a cross-section in longitudinal direction through the injection syringe when the first reservoir is almost empty.

In the drawing the same or similar elements carry the same reference numerals.

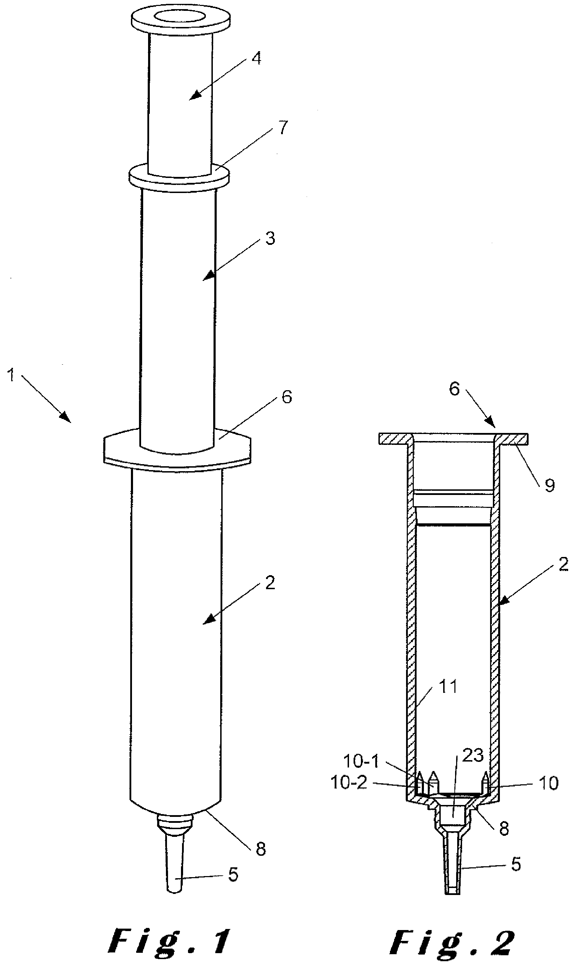

Figure 1 shows an overall view of an injection syringe, in particular an intramammary injection syringe, according to the invention. The injection syringe 1 is formed of three parts and comprises a first reservoir 2 provided with a first open end 6 through which the second reservoir 3 can be mounted sliding. For this the diameter of the second reservoir is slightly smaller than the internal diameter of the first reservoir. The second reservoir also comprises an open end 7 through which a piston 4 can be mounted sliding. At a second end 8 of the first reservoir 2, which second end is located opposite the first end 6, an injection needle 5 is arranged. The latter is preferably made of one piece with the reservoir since this offers a cheap and reliable solution which in addition ensures that no leakage can occur between the first reservoir and the injection needle.

The injection syringe is preferably made of plastic by injection moulding. Furthermore the first and second reservoir and the piston are preferably substantially cylindrical, as this forms an injection syringe which is comfortable to handle and in addition simple to produce.

In the first reservoir 2 is preferably stored a drug or other medication substance, while the second reservoir 3 normally contains a salve as a substance. The injection syringe is preferably intended for veterinary medicine, in particular for the administration of antibiotics to the udder of a cow. The antibiotics must not mix with the salve in the injection syringe and therefore it is important that no leakage can occur from the second to the first reservoir.

Figure 2 shows a cross-section in longitudinal direction through the first reservoir 2. The first reservoir is fitted with a finger grip 9 arranged around the first open end 6. As the word implies, the finger grip serves for pressing of two fingers on operation of the injection syringe. At the second end 8 on the inner casing 11 of the first reservoir 2 is arranged a bulge 10. Preferably the bulge 10 is formed by standing ribs. The standing ribs have a height of between 4 and 6 mm, preferably 5 mm, and are formed of one piece with the inner casing of the first reservoir. The ribs have a thickness of 1 to 2 mm. Preferably the standing ribs are arranged in pairs in a star shape (10-1, 10-2).

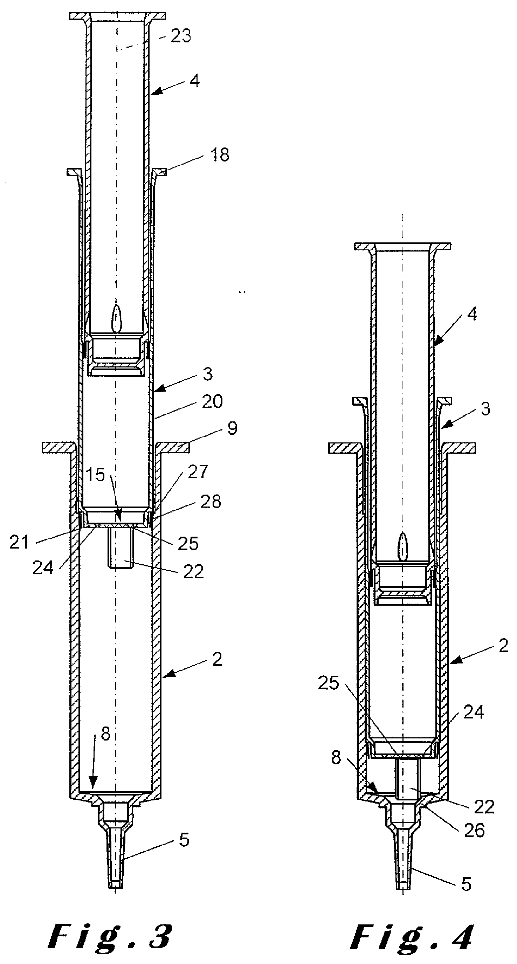

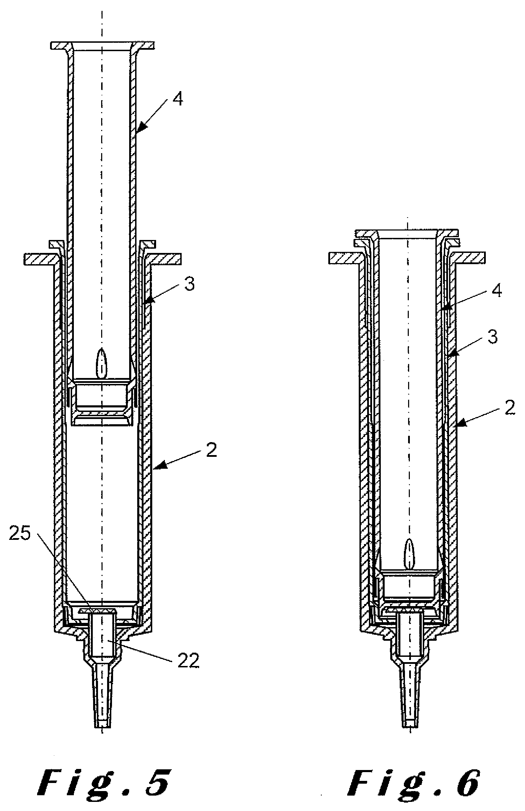

Figure 3 shows a cross-section in longitudinal direction through the injection syringe wherein the first 2 and the second reservoir 3 are filled. The second reservoir at the upper edge is fitted with a stop 18 which is intended to run against the finger grip 9 of the first reservoir when the second reservoir is fully inserted in the first, as shown in figure 5. The second reservoir furthermore has a casing surface 20 which transforms into a base surface 21. At a lower edge of the base surface is arranged an outlet 15 of the second reservoir. Arranged on this outlet is a protrusion 22. In the mounted state of the injection syringe, the protrusion extends from the base surface 21 of the second reservoir in the direction towards the second end 8 of the first reservoir. The protrusion is arranged breakably on the base surface of the second reservoir, such that on reaching the second end of the first reservoir, the protrusion breaks off to expose the outlet of the second reservoir.

As shown in figures 3 and 4, the protrusion is offset in relation to a central axis 23 of the injection syringe. Furthermore the protrusion preferably has a Y-shaped profile in cross-section. In the base surface 21 of the second reservoir is formed a circular weakness 24 which delimits a disc 25 on which the protrusion 22 is arranged.

On use of the injection syringe according to the invention, for example for injecting an antibiotic into the udder of a cow, the user first gradually presses the second reservoir into the first reservoir as shown in figure 3. Thereby the substance from the first reservoir 2 emerges through the injection needle 5 and is injected into the udder of the cow. As the second reservoir is pressed deeper into the first reservoir, the latter empties more and more.

When the protrusion 22 of the second reservoir 3 approaches the second end 8 of the first reservoir as shown in figure 4, the protrusion 22 on the base surface 21 of the second reservoir 3 comes into contact with the second end 8 of the first reservoir. As a result the protrusion 22 comes into contact with the peripheral edge 26 on the inside of the base surface 8 of the first reservoir. As the second reservoir is pressed further into the first, the pressure exerted on the protrusion increases, whereby the latter bends away as it catches on the peripheral wall. Due to the presence of the circular weakness 24 in the base surface and the pressure exerted on the protrusion, the disc 25 breaks away along the weakening whereby the outlet from the second reservoir is opened and the protrusion can penetrate the second reservoir. Now the substance can escape from the second reservoir and flow via the outlet of the first reservoir in the direction of the needle 5 and be administered.

The position of the protrusion offset in relation to the central axis 23 offers the advantage that the protrusion 22 first makes contact with the edge of the outlet 8 whereby not only is a greater force exerted on the protrusion but it is also exerted slightly earlier than if the protrusion were mounted centrally. If in addition the protrusion has a Y-shaped profile, this increases the transfer force to the weakening since the protrusion is effectively twisted and the force is bundled.

As shown in figure 5, when both reservoirs are empty, the disc 25 is completely broken off and the protrusion sits firmly in the outlet of the first reservoir, whereby both reservoirs are now closed.

The second reservoir furthermore comprises a sealing element 27 which is formed by a flexible ring mounted on the casing surface of the second reservoir. The sealing element is arranged close to the base surface in order in this way to obtain a scraping effect along the inner casing of the first reservoir. Furthermore below the sealing element is preferably arranged a skirt 28 which creates the necessary flexibility when the second reservoir is pressed inward.

## Claims

1. Injection syringe (1), in particular an intramammary injection syringe, comprising a first and a second reservoir (2,3), which first reservoir (2) has a first open end (6) through which the second reservoir (3) can be mounted sliding in the first reservoir (2), which first reservoir (2) has a second end (8) located opposite the first end (6) and on which an injection needle (5) can be arranged, which second reservoir (3) comprises a base surface which is fitted with an outlet, **characterised in that** at the outlet is arranged a protrusion (22) which in mounted state of the injection syringe (1) extends from the base surface (21) in the direction of the second end (8), which protrusion (22) is arranged breakably on the base surface of the second reservoir (3) and which protrusion (22) is arranged offset in relation to a central axis (23) of the injection syringe (1), such that when the protrusion (22) reaches the second end (8) it breaks away to open the outlet of the second reservoir (3).

2. injection syringe according to claim 1, **characterised in that** the protrusion (22) has a Y-shaped profile in cross-section.

3. injection syringe according to claim 1 or 2, **characterised in that** in the base surface is arranged a circular weakness which delimits a disc (25) on which the protrusion (22) is arranged.

4. injection syringe according to any of claims 1 to 3, **characterised in that** the first and the second reservoir (2,3) are substantially cylindrical and that the second reservoir (3) is provided with a sealing element which is formed by a flexible ring arranged along a circumference of the casing surface of the second reservoir (3).

5. Injection syringe according to any of claims 1 to 4, **characterised in that** the base surface is ringed by a skirt.

6. injection syringe according to claims 4 and 5, **characterised in that** the skirt extends below the sealing element.

## Patentansprüche

1. Injektionsspritze (1), insbesondere eine intramammäre Injektionsspritze, umfassend ein erstes und ein zweites Reservoir (2,3), von denen das erste Reservoir (2) ein erstes offenes Ende (6) hat, durch das das zweite Reservoir (3) durch Einschieben in das erste Reservoir (2) montiert werden kann, das erste Reservoir (2) hat ein dem ersten Ende (6) gegenüberliegendes zweites Ende (8), an dem eine Injektionsnadel (5) angeordnet werden kann, das zweite Reservoir (3) umfasst eine mit einem Auslass versehene Grundfläche, **dadurch gekennzeichnet, dass** an dem Auslass ein Vorsprung (22) angeordnet ist, der sich im montierten Zustand der Injektionsspritze (1) von der Grundfläche (21) in Richtung des zweiten Endes (8) erstreckt, der Vorsprung (22) ist abbrechbar auf der Grundfläche des zweiten Reservoirs (3) angeordnet und der Vorsprung (22) ist versetzt im Verhältnis zur Mittelachse (23) der Injektionsspritze (1) angeordnet, so dass, wenn der Vorsprung (22) das zweite Ende (8) erreicht, wegbricht um den Auslass des zweiten Reservoirs (3) zu öffnen.

2. Injektionsspritze nach Anspruch 1, **dadurch gekennzeichnet, dass** der Vorsprung (22) im Querschnitt ein Y-förmiges Profil hat.

3. Injektionsspritze nach Anspruch 1 oder 2, **dadurch gekennzeichnet, dass** in der Grundfläche eine kreisförmige Schwachstelle angeordnet ist, die eine Scheibe (25) begrenzt, auf der der Vorsprung (22) angeordnet ist.

4. Injektionsspritze nach einem der Ansprüche 1 bis 3, **dadurch gekennzeichnet, dass** das erste und das zweite Reservoir (2,3) im Wesentlichen zylindrisch sind und dass das zweite Reservoir (3) mit einem Dichtelement versehen ist, das durch einen flexiblen Ring gebildet wird, der entlang eines Umfangs der Gehäusefläche des zweiten Reservoirs (3) angeordnet ist.

5. Injektionsspritze nach einem der Ansprüche 1 bis 4, **dadurch gekennzeichnet, dass** die Grundfläche von einem Mantel umgeben ist.

6. Injektionsspritze nach einem der Ansprüche 4 und 5, **dadurch gekennzeichnet, dass** der Mantel sich unter das Dichtelement erstreckt.

## Revendications

1. Seringue d'injection (1), en particulier une seringue d'injection intramammaire, comprenant un premier et un deuxième réservoir (2,3), ledit premier réservoir (2) comprenant une première extrémité ouverte (6) à travers laquelle le deuxième réservoir (3) peut être monté de façon coulissante dans le premier réservoir (2), ledit premier réservoir (2) comprenant une deuxième extrémité (8) située à l'opposé de la première extrémité (6) et sur laquelle une aiguille d'injection (5) peut être agencée, ledit deuxième réservoir (3) comprenant une surface de base qui est pourvue d'une sortie, **caractérisée en ce qu'**une saillie (22) est disposée à la sortie, qui, dans un état monté de la seringue d'injection (1), s'étend à partir de la surface de base (21) dans la direction de la deuxième extrémité (8), ladite saillie (22) étant agencée de façon cassable sur la surface de base du deuxième réservoir (3), et ladite saillie (22) étant disposée de façon décalée par rapport à un axe central (23) de la seringue d'injection (1), de telle sorte que lorsque la saillie (22) atteint la deuxième extrémité (8), elle se casse pour ouvrir la sortie du deuxième réservoir (3).

2. Seringue d'injection selon la revendication 1, **caractérisée en ce que** la saillie (22) comprend un profil en forme de Yen section transversale.

3. Seringue d'injection selon la revendication 1 ou 2, **caractérisée en ce qu'**une zone affaiblie circulaire est agencée dans la surface de base et qui délimite un disque (25) sur lequel la saillie (22) est formée.

4. Seringue d'injection selon l'une quelconque des revendications 1 à 3, **caractérisée en ce que** le premier et le deuxième réservoir (2,3) sont essentiellement cylindriques, et **en ce que** le deuxième réservoir (3) est pourvu d'un élément d'étanchéité qui est formé par un anneau flexible agencé le long d'une circonférence de la surface d'enceinte du deuxième réservoir (3).

5. Seringue d'injection selon l'une quelconque des revendications 1 à 4, **caractérisée en ce que** la surface de base est annelée par une jupe.

6. Seringue d'injection selon les revendications 4 et 5, **caractérisée en ce que** la jupe s'étend en dessous de l'élément d'étanchéité.
